# EUROPEAN PATENT SPECIFICATION

(11) **EP 2 915 591 B1**
(45) Date of publication and mention of the grant of the patent: **01.02.2017**
(21) Application number: 13851491.4
(22) Date of filing: 31.10.2013
(51) Int. Cl.: B05B 17/04, B05B 7/22, C23C 4/10, C23C 4/11, C23C 4/134, C23C 4/02, G01N 33/00

(54) **OXYGEN SENSOR HAVING POROUS CERAMIC COATING LAYER FORMED THEREON AND METHOD FOR FORMING POROUS CERAMIC COATING LAYER**
SAUERSTOFFSENSOR MIT PORÖSER KERAMIKBESCHICHTUNG DARAUF UND VERFAHREN ZUR HERSTELLUNG EINERN PORÖSEN KERAMIKBESCHICHTUNG
CAPTEUR D'OXYGÈNE AYANT UNE COUCHE DE REVÊTEMENT DE CÉRAMIQUE POREUSE FORMÉE SUR CELUI-CI ET PROCÉDÉ DE FORMATION DE COUCHE DE REVÊTEMENT DE CÉRAMIQUE POREUSE

(30) Priority: 31.10.2012 KR 20120122600
(43) Date of publication of application: 09.09.2015
(73) Proprietor: Hyundai Kefico Corporation, Gunpo-si, Gyeonggi-do 435-716 (KR)
(72) Inventor: CHUNG, Yun-Ki, Seoul 151-015 (KR); KIM, Jung-Taek, Gunpo-si Gyeonggi-do 435-716 (KR); LEE, Dae-Gun, Gunpo-si Gyeonggi-do 435-716 (KR); KO, Yang-Joo, Seongnam-si Gyeonggi-do 463-972 (KR)
(74) Representative: Cabinet Laurent & Charras
(86) International application number: PCT/KR2013/009792
(87) International publication number: WO 2014/069915

(56) References cited:
- EP-A1- 1 942 387
- EP-A2- 1 109 013
- WO-A2-2008/151054
- JP-A- H1 019 836
- JP-A- 2001 099 806
- JP-A- 2002 323 473
- JP-A- 2003 322 632
- JP-A- 2004 066 104
- Sulzer Metco: "Thermal Spray Equipment Guide", , 1 January 2010 (2010-01-01), XP055260460, Retrieved from the Internet: URL:http://www.jya.cl/wp-content/uploads/2 013/02/Equipment_Guide_EN8_web.pdf [retrieved on 2016-03-22]
- SARAFOGLOU ET AL: "Study of Al2O3 coatings on AISI 316 stainless steel obtained by controlled atmosphere plasma spraying (CAPS)", SURFACE AND COATINGS TECHNOLOGY, ELSEVIER, AMSTERDAM, NL, vol. 202, no. 1, 9 October 2007 (2007-10-09), pages 155-161, XP022290097, ISSN: 0257-8972, DOI: 10.1016/J.SURFCOAT.2007.05.021

## Description

This application claims the benefit of Korean Patent Application No. 10-2012-0122600, filed on October 31, 2012.

### Technical Field

The present invention relates to an oxygen sensor including a porous ceramic coating layer and a method of forming a porous ceramic coating layer. More particularly, the present invention relates to an oxygen sensor including a porous ceramic coating layer formed on the surface of a sensing part of the oxygen sensor by plasma-coating the sensing part with ceramic powder having a predetermined particle size, and a method of forming a porous ceramic coating layer.

### Background Art

Generally, an oxygen sensor is mounted at a predetermined position of an exhaust manifold and detects the concentration of oxygen included in exhaust gas to allow an engine control unit (ECU) to perform feedback for air-fuel ratio control (lambda value) using the detected oxygen concentration.

Such an oxygen sensor is manufactured in consideration of temperature limit with respect to each part thereof. That is, the temperature limit for a header part thereof is set to about 650°C, the temperature limit for a grommet part thereof is set to about 200 °C, and the temperature limit for a tip part thereof is set to about 900 °C.

Since the header part of the oxygen sensor is directly in contact with an exhaust manifold, it is closely related to the temperature of the surface of the exhaust manifold. The grommet part of the oxygen sensor is closely related to the temperature of the periphery of the exhaust manifold, the periphery being provided with the oxygen sensor.

An oxygen sensor does not cause a problem when an engine is placed in a stationary state, that is, when an intake manifold is located near a radiator and an exhaust manifold is located near a driver (dash panel). However, as an intake-exhaust-reversed engine, in which an intake manifold is located near a driver and an exhaust manifold is located near a radiator, is developed, the temperature of the oxygen sensor exceeds the temperature limit thereof, thus causing a problem of thermally damaging the oxygen sensor.

Further, the temperature limit of exhaust gas was increased to 830 ∼ 850 °C in order to stabilize the emission of exhaust gas, but the problem of thermally damaging the oxygen sensor has not been solved yet.

In the intake-exhaust-reversed engine, thermally-damaged parts include a main harness, a plastic head cover, a head cover gasket, etc., which are located near an exhaust manifold, in addition to an oxygen sensor.

When the temperature of a header part of an oxygen sensor exceeds the temperature limit thereof, the durable diaphragm in the oxygen sensor becomes opened, and thus intake gas and exhaust gas are mixed, thereby causing a phenomenon of output signals of the oxygen sensor disappearing.

Further, when the temperature of a grommet part of an oxygen sensor exceeds the temperature limit thereof, a sealing rubber surrounding the ends of four wires of the oxygen sensor is melted, so water permeates thereinto, and thus the oxygen sensor is corroded, thereby causing a wire short.

In order to prevent the above phenomenon, a method of blocking the radiation heat of an exhaust manifold by surrounding the oxygen sensor with a heat protector has been proposed. However, this method is also problematic in that the shape of the heat protector is not configured to surround the entire oxygen sensor, so the heat protector cannot completely block the radiation heat of the exhaust manifold, and thus the thermal damage of the oxygen sensor cannot be completely prevented.

Further, this method is problematic in that the heat protector inhibits air from smoothly flowing around the oxygen sensor, and thus the radiation heat of the exhaust manifold stays in the heat protector, thereby increasing the thermal damage of the oxygen sensor.

As a conventional technology related thereto, there is Korean Patent Application Publication No. 1998-0038809 (Apparatus for inspecting the deterioration of oxygen sensor and method of controlling the operation thereof, published on August 17, 1998).

### Disclosure

### Technical Problem

An object of the present invention is to provide an oxygen sensor, wherein its sensing part is provided with a porous ceramic coating layer, thus preventing the sensing part from being damaged by the thermal shock from exhaust gas occurring when water particles formed by the difference in temperature between the inside and outside of exhaust gas come into contact with the sensing part of the oxygen sensor.

Another object of the present invention is to provide an oxygen sensor, wherein the durability of the oxygen sensor to thermal shock is improved, thus allowing an internal combustion engine to be maintained at an approximately ideal air-fuel ratio.

Still another object of the present invention is to provide an oxygen sensor, wherein it is possible to prevent the sensing part of the oxygen sensor from being poisoned by harmful materials in exhaust gas, thus allowing the feedback of ECU to be smoothly performed by the oxygen sensor.

### Technical Solution

In order to accomplish the above objects, an oxygen sensor coating apparatus is provided, which includes: a linear guide provided over the ground; a plasma gun connected to the guide and spraying a coating material containing ceramic powder onto an oxygen sensor as an object material using a plasma flame while moving straight along the guide; a reaction gas injector injecting reaction gas onto the object material while moving straight along the guide together with the plasma gun; and an object material fixing module provided opposite to the plasma gun and fixing the object material.

The object material fixing module may include: a base frame which is supported on the ground; a connecting arm, one end of which is connected to the base frame; and a jig provided with a fixing bracket for fixing the object material and connected to the other end of the connecting arm to be rotated by driving the connecting arm.

The object material may be an oxygen sensor.

The coating material may be ceramic powder.

The plurality of fixing brackets may be provided along one side of the jig.

The fixing bracket may be rotatably provided on one side of the jig.

The plasma gun may move in the direction of arrangement of the fixing brackets.

The plasma gun is characterized in that it moves in the direction of arrangement of the fixing brackets, and the fixing bracket for fixing the object material rotates at predetermined angle intervals with respect to each coating according to the movement of the plasma gun.

When the fixing bracket rotates to an angle of 360°, the connecting arm may rotate the jig at predetermined angle intervals based on the end (serving as a rotation axis) of the object material fixed in the fixing bracket, and simultaneously plasma coating may be performed.

When the jig rotates at predetermined angle intervals to allow the fixing bracket to be flush with the plasma gun, the fixing bracket may not rotate, and the plasma gun may perform plasma coating once.

The reaction gas may be selected from among O₂, O₃, N₂, H₂, Ar and combinations thereof, and the coating material may be selected from among: oxide powder including Y₂O₃, Al₂O₃, ZrO₂, TiO₂ and SiO₂; nitride powder including AlN, Ti and BN; carbide powder including SiC and AlC; and combinations thereof.

The ceramic powder sprayed through the plasma gun has a particle diameter of 20 to 150 µm.

The ceramic coating layer applied on the object material may have a porosity of 10 to 50%.

The ceramic coating layer may be immersed in a platinum (Pt) solution.

The platinum (Pt) may be uniformly distributed in the ceramic coating layer in an amount of 0.3 to 5.0%.

The present invention provides a method of forming a porous ceramic coating layer on an oxygen sensor, including the steps of: fixing an oxygen sensor in any one of a plurality of fixing brackets provided along one side of a jig; spraying metal powder onto a sensing part of the oxygen sensor using a plasma gun and simultaneously injecting reaction gas onto the sensing part through a reaction gas injector to form an adhesion layer; injecting gas into the plasma gun provided with a positive electrode and a negative electrode through a gas inlet and then applying high voltage to the positive electrode and the negative electrode to generate a plasma flame; and spraying ceramic powder into the plasma flame through a powder inlet provided at the plasma gun to form a porous ceramic coating layer on the adhesion layer.

The step of forming the porous ceramic coating layer may include the steps of: a) forming the porous ceramic coating layer on the adhesion layer while moving the plasma gun straight to the sensing part of the oxygen sensor fixed in the fixing bracket along one side of the jig; and b) repeatedly performing step a) while rotating the fixing bracket at predetermined angle intervals after step a).

The step of forming the porous ceramic coating layer may further include the steps of: c) rotating the jig at predetermined angle intervals based on the sensing part (rotation axis) of the oxygen sensor fixed in the fixing bracket, when the fixing bracket rotates to an angle of 360° in the step b); d) repeatedly performing steps a) and b) after step c); and e) allowing the fixing bracket not to rotate, repeatedly performing step a) and then finishing the step of forming the porous ceramic coating layer, when the rotation axis of the fixing bracket of the jig is flush with the plasma gun after step d).

In the step of forming the adhesion layer, the reaction gas may be selected from among O₂, O₃, N₂, H₂, Ar and combinations thereof, and, in the step of forming the porous ceramic coating layer, the ceramic powder may be selected from among: oxide powder including Y₂O₃, Al₂O₃, ZrO₂, TiO₂ and SiO₂; nitride powder including AlN, Ti and BN; carbide powder including SiC and AlC; and combinations thereof.

In the step of forming the adhesion layer, the adhesion layer may have a thickness of 0.1 to 10 µm.

In the step of forming the porous ceramic coating layer, the ceramic powder sprayed through the plasma gun may have a particle diameter of 20 to 150 µm.

In the step of forming the porous ceramic coating layer, the porous ceramic coating layer may have a thickness of 200 to 500 µm.

In the step of forming the porous ceramic coating layer, the porous ceramic coating layer may have a porosity of 10 to 50%.

The ceramic coating layer may be immersed in a platinum (Pt) solution.

The platinum (Pt) may be uniformly distributed in the ceramic coating layer in an amount of 0.3 to 5.0%.

### Advantageous Effects

According to the oxygen sensor of the present invention, its sensing part is provided with a porous ceramic coating layer, thus preventing the sensing part from being damaged by the thermal shock from exhaust gas.

Further, according to the oxygen sensor of the present invention, the durability of the oxygen sensor to thermal shock is improved, thus allowing an internal combustion engine to be maintained at an approximately ideal air-fuel ratio.

Further, according to the oxygen sensor of the present invention, it is possible to prevent the sensing part of the oxygen sensor from being poisoned by harmful materials in exhaust gas, thus allowing the feedback of ECU to be smoothly performed by the oxygen sensor.

### Description of Drawings

FIG. is a schematic view showing a ceramic coating apparatus.
FIG. 2 is a schematic view showing the ceramic coating apparatus, which was rotated at an angle of 45°.
FIG. 3 is a schematic view showing the ceramic coating apparatus, which was rotated at an angle of 90°.
FIG. 4 is a schematic perspective view showing a jig.
FIG. 5A to 5D are schematic views showing oxygen sensors according to the steps a) and b) in the method of forming a porous ceramic coating layer according to the present invention.
FIG. 6A to 6D are schematic views showing oxygen sensors according to the steps c) and d) in the method of forming a porous ceramic coating layer according to the present invention.
FIG. 7 is a schematic view showing an oxygen sensor according to the step e) in the method of forming a porous ceramic coating layer according to the present invention.
FIG. 8 is a schematic block diagram showing a method of forming a porous ceramic coating layer on an oxygen sensor according to the present invention.
FIG. 9 is a schematic block diagram showing the steps of forming a porous ceramic coating layer on an oxygen sensor according to the present invention.
FIG. 10 is a schematic sectional view showing an oxygen sensor including a porous ceramic coating layer, which was formed by the method according to the present invention.
FIG. 11 is an enlarged view of the porous ceramic coating layer of the present invention, which was enlarged by a magnifying power of 339.
FIG. 12 is an enlarged view of the porous ceramic coating layer of the present invention, which was enlarged by a magnifying power of 189.
FIG. 13 is an enlarged view of the porous ceramic coating layer of the present invention, which was enlarged by a magnifying power of 493.
FIG. 14 is an enlarged view of the porous ceramic coating layer of the present invention, which was enlarged by a magnifying power of 2.48k.

### Best Mode

Hereinafter, preferred embodiments of the present invention will be described in detail with reference to the attached drawings.

Here, when it is determined that the detailed description of the related art would obscure the gist of the present invention, the description thereof will be omitted.

Further, preferred embodiments of the present invention are provided in order to completely explain the present invention to those skilled in the art.

Therefore, the shapes and sizes of the elements in the drawings may be exaggerated for clear explanations.

Plasma coating is referred to as flame spray coating including the steps of generating a plasma flame (arc) and supplying various kinds of coating material powders to the plasma flame.

Examples of flame spray coating may include flame wire spray coating, flame powder spray coating, high-velocity flame powder spray coating, high-velocity flame wire spray coating, flame rod spray coating, electric arc wire spray coating, plasma powder spray coating, and the like.

In the present invention, the oxygen sensor 200 is referred to as a sensor for detecting the concentration of oxygen included in exhaust gas of an automobile and then transmitting the detected value to an engine control unit (ECU) to allow the injection ratio of fuel to be approximate to an air-fuel ratio.

The apparatus for coating an oxygen sensor 200 with porous ceramics will be described as follows.

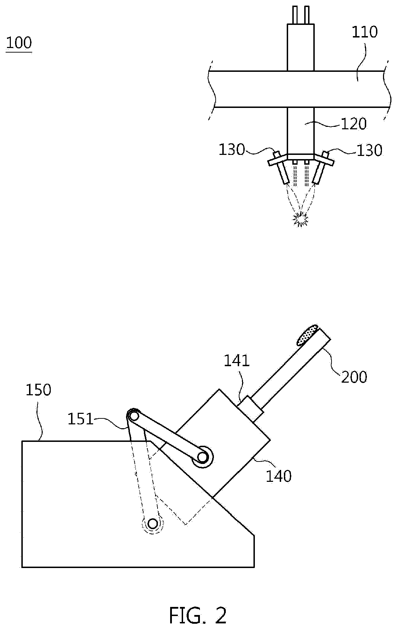

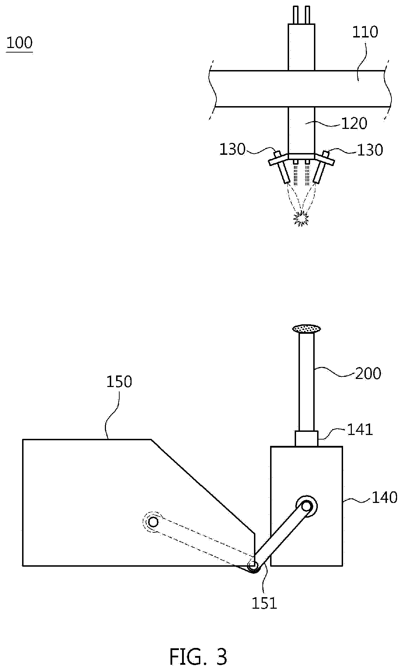

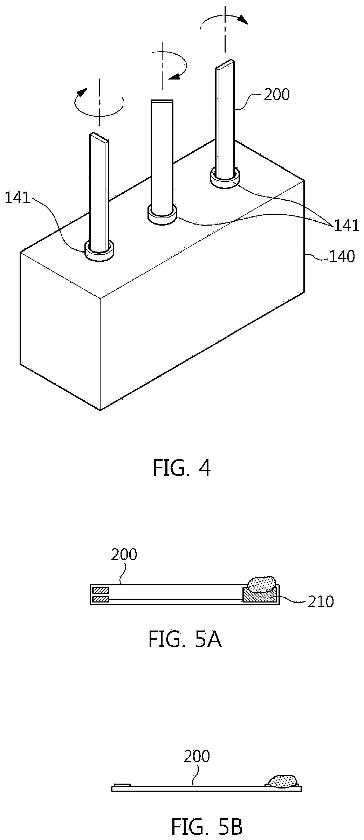

FIG. is a schematic view showing a ceramic coating apparatus, FIG. 2 is a schematic view showing the ceramic coating apparatus, which was rotated at an angle of 45°, FIG. 3 is a schematic view showing the ceramic coating apparatus, which was rotated at an angle of 90°, and FIG. 4 is a schematic perspective view showing a jig according to the present invention.

The ceramic coating apparatus 100 for an oxygen sensor 200 includes: a linear guide 110 provided over the ground; a plasma gun 120 connected to the guide 110 and spraying a coating material onto an object material using plasma flame while moving straight along the guide 110; a reaction gas injector 130 injecting reaction gas onto the object material while moving straight along the guide 110 together with the plasma gun 120; a jig 140 provided opposite to the plasma gun 120 and including a fixing bracket 141 for fixing the object material; and a base frame 150 including a connecting arm 151 for connecting the jig 140 with a body provided on the ground and allowing the jig 140 to be rotated by driving the connecting arm 151.

The guide 110 is provided over the ground. In this case, the guide 110 may be provided in a box for plasma coating.

The guide 110 may be used as a pair of guides 110 spaced apart from each other. The pair of guides 110 are provided with grooves in a direction opposite to each other, and the plasma gun 120 is connected to the grooves of the guides to move straight along the guides 110.

The plasma gun 120 is provided therein with a positive electrode and a negative electrode, and high voltage is applied to the positive electrode and the negative electrode. Further, the plasma gun 120 is provided therein with a reaction gas injector 130 for injecting reaction gas. The plasma gun generates a plasma flame using the high voltage applied to the positive electrode and the negative electrode.

Metal powder or ceramic powder, as a coating material, is supplied to the plasma flame to perform plasma spray coating.

Since the plasma gun 120 is commonly known, a detailed description thereof will be omitted.

The guide 110 is provided thereunder with a base frame 150 provided on the ground and a jig 140 connected to the base frame 150.

The body of the base frame 150 is strongly fixed on the ground. Preferably, the base frame 150 may be provided on the bottom of a box provided with the guide 110.

The connecting arm 151 may consist of a plurality of joint links or an extensible or retractable cylinder, and may be operated to rotate the jig 140.

It is preferred that a pair of connecting arms 151 be proved at both sides of the jig 140.

The jig 140 is provided on one side thereof with a fixing bracket 141 for fixing the oxygen sensor 200.

A plurality of fixing brackets 141 are provided on one side of the jig 140 in the length direction of the jig 140. As shown in FIG. 2, the fixing bracket 141 is rotatably provided.

In the present invention, the fixing bracket 141 is configured to rotate at 90° intervals, but the rotation range or interval of the fixing bracket 141 is not limited.

Meanwhile, the jig 140 rotates based on an imaginary line (serving as a rotation axis) that links the sensing parts 210 of the oxygen sensors 200 fixed in the fixing brackets 141, that is, the ends (sensing parts 210) of the oxygen sensors respectively fixed in the fixing brackets 141 provided along ones side of the jig 140.

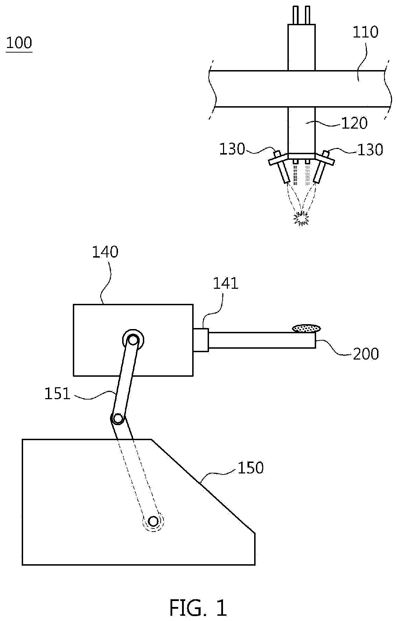

In the present invention, the jig 140 rotates at 45° intervals. First, as shown in FIG. 1, plasma spray coating is performed in a direction parallel to the ground, and is then performed at an angle of 45°, and, finally, is performed in a direction perpendicular to the ground.

In the present invention, the reaction gas injected from the reaction gas injector 130 may be selected from among O₂, O₃, N₂, H₂, Ar and combinations thereof, and the coating material applied to the object material (oxygen sensor 200) may be selected from among: oxide powder including Y₂O₃, Al₂O₃, ZrO₂, TiO₂ and SiO₂; nitride powder including AlN, Ti and BN; carbide powder including SiC and AlC; and combinations thereof.

Particularly, the ceramic powder sprayed through the plasma gun 120 has a particle diameter of 20 to 150 µm, and the ceramic coating layer 230 formed on the object material (oxygen sensor 200) by plasma spray coating is a porous ceramic coating layer having a porosity of 10 to 50%.

Meanwhile, the ceramic coating layer 230 is immersed in a platinum (Pt) solution such that platinum is uniformly distributed in the ceramic coating layer 230 in an amount of 0.5 to 5.0%.

The platinum (Pt) is uniformly distributed in the ceramic coating layer 230 to serves as a catalyst.

As such, when the porous ceramic coating layer 230 is formed on the sensing part 210 of the oxygen sensor 200, the heat of exhaust gas is not rapidly transferred to the sensing part 210, thus preventing the oxygen sensor 200 from being damaged by the heat.

Hereinafter, a method of forming a porous ceramic coating layer 230 on an oxygen sensor 200 according to the present invention will be described as follows.

The method of forming a porous ceramic coating layer 230 on an oxygen sensor 200 according to the present invention includes the steps of: fixing an oxygen sensor 200 in any one of a plurality of fixing brackets 141 provided along one side of a jig 140 (S100); spraying metal powder onto a sensing part 210 of the oxygen sensor 200 using a plasma gun 120 and simultaneously injecting reaction gas onto the sensing part 210 through a reaction gas injector 130 to form an adhesion layer 220 (S200); injecting gas into the plasma gun 120 provided with a positive electrode and a negative electrode through a gas inlet and then applying high voltage to the positive electrode and the negative electrode to generate a plasma flame (S300); and spraying ceramic powder into the plasma flame through a powder inlet provided at the plasma gun 120 to form a porous ceramic coating layer 230 on the adhesion layer 220 (S400).

First, as described above, an oxygen sensor 200 is inserted and fixed in any one of a plurality of fixing brackets 141 provided at regular intervals along one side of a jig 140 (S100). The fixing bracket 141 is provided such that it rotates at 90° intervals. The fixing bracket 141 may be provided with a hole for inserting the oxygen sensor 200.

Subsequently, an adhesion layer 220 is formed between a sensing part 210 of the oxygen sensor 200 and a porous ceramic coating layer 230 to be applied on the sensing part 210 such that the porous ceramic coating layer 230 is easily attached to the surface of the oxygen sensor 200 S200).

Specifically, when metal powder containing a ceramic material is sprayed onto the sensing part 210 of the oxygen sensor 200 through a powder inlet provided in a plasma gun 120, and simultaneously reaction gas (a combination of oxide gas, nitride gas and carbide gas, more specifically, a combination of O₂, O₃, N₂, H₂ and Ar) is injected onto the sensing part 210 through a reaction gas injector 130, the reaction gas reacts with the sprayed metal powder, thus forming the adhesion layer 220 on the surface of the sensing part 210 of the oxygen sensor 200.

Here, the kind of metal powder is not limited as long as it can obtain the same effect.

The plasma gun 120 moves straight along the guide 110, That is, the plasma gun 120 moves along the sensing parts 210 of a plurality of oxygen sensors 200 fixed in one side of the jig 140.

That is, the plasma gun 120 moves to the sensing part 210 of the oxygen sensor 200 fixed in the fixing bracket 141 along the guide 110 in the direction of arrangement of the fixing brackets 141 of the jig 140 to form the adhesion layer 220 on the surface of the sensing part 210 of the oxygen sensor 200.

The metal powder is sprayed such that the thickness of the adhesion layer is 0.1 to 10 µm.

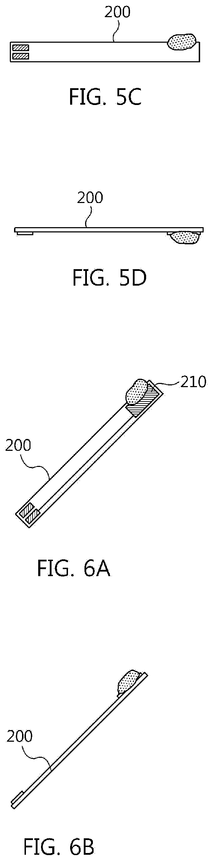

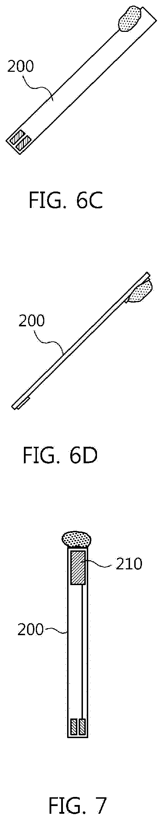

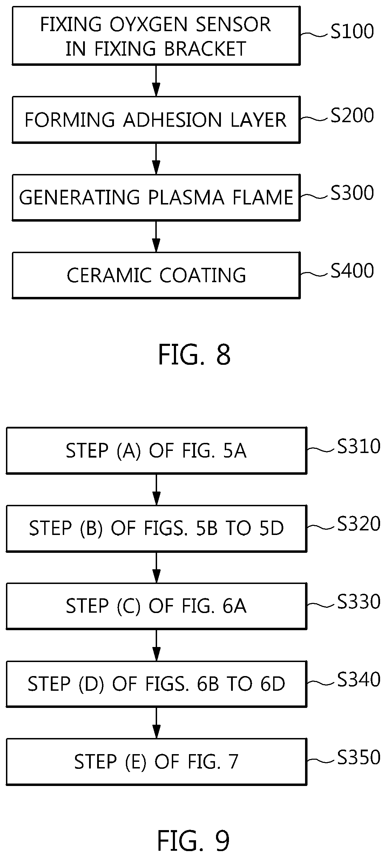

Next, the step (S400) of forming the porous ceramic coating layer 230 will be described with reference to FIGS. 1 to 9.

In the step (S200) of forming the adhesion layer 220, metal powder is introduced through a powder inlet, but, in the step (S400) of forming the porous ceramic coating layer 230, ceramic powder is introduced through the powder inlet.

In the present invention, ceramic powder having a particle diameter of 20 to 150 µm is used.

When ceramic powder is introduced, high voltage is applied to the positive electrode and negative electrode provided in the plasma gun 120 and the reaction gas is injected through the reaction gas injector 130, a plasma flame is generated. Then, when ceramic powder is supplied to this plasma flame, the porous ceramic coating layer 230 is formed on the surface of the sensing part 210 of the oxygen sensor 200.

Here, the ceramic powder may be selected from among: oxide powder including Y₂O₃, Al₂O₃, ZrO₂, TiO₂ and SiO₂; nitride powder including AlN, Ti and BN; carbide powder including SiC and AlC; and combinations thereof.

Meanwhile, the ceramic coating layer 230 formed by the method of the present invention is immersed in a platinum (Pt) solution such that platinum is uniformly distributed in the ceramic coating layer 230 in an amount of 0.5 to 5.0%.

The plasma gun 120 moves straight to the oxygen sensor 200 fixed in the fixing bracket 141 along one side of the jig 140, thus forming a porous ceramic coating layer 230 on the adhesion layer 220 (S310).

When three oxygen sensors 200 are fixed on one side of the jig 140, ceramic coating layers 230 are formed by the plasma gun 120 and then each of the oxygen sensors 200 is plasma-coated once, the fixing bracket 141 rotates at an angle of 90°, and, as described above, the procedure of forming the ceramic coating layer 230 is performed once more.

That is, in the oxygen sensor 200 having a rectangular parallelepiped, when a ceramic coating layer 230 is formed on one side of the oxygen sensor, at which the sensing part 210 is located, in order to perform plasma coating over the entire surface of one end of the oxygen sensor 200, the fixing bracket 141 rotates at 90° intervals to perform the plasma coating even on another side thereof (S320).

When the fixing bracket 141 rotates at 90° intervals to finally rotate to an angle of 360°, the jig 140 is inclined at an angle of 45° by the connecting arm 151. In this case, the jig 140 rotates based on the imaginary rotation axis connecting the sensing parts 210 of the plurality of oxygen sensors 200 fixed on one side of the jig 140, thus preventing the sensing parts 210 from deviating from the movement line of the plasma gun 120.

When the jig 140 is rotated or inclined at an angle of 45°, the plasma gun 120 moves straight, and simultaneously a ceramic coating layer is formed (S330). As described above, when the ceramic coating layer 230 is formed on one side of the oxygen sensor, the fixing bracket 141 rotates at 90° intervals to perform the plasma coating even on another side thereof (S340).

When the fixing bracket 141 rotates at 90° intervals to finally rotate to an angle of 360°, the fixing bracket 141 is further rotated or inclined at an angle of 45°, so the jig 140 is rotated to an angle of 90° based on the initial position thereof, and thus the rotation axis of the fixing bracket 141 is flush with the plasma gun 120 to allow the upper end of the oxygen sensor 200 to face the plasma gun 120.

In this state, ceramic powder is sprayed by the plasma gun 120, thus finishing the formation of the ceramic coating layer 230 (S350).

The ceramic powder is sprayed such that the thickness of the ceramic coating layer is 200 to 500 µm. The ceramic coating layer 230 formed in this way is a porous ceramic coating layer having a porosity of 10 to 50%.

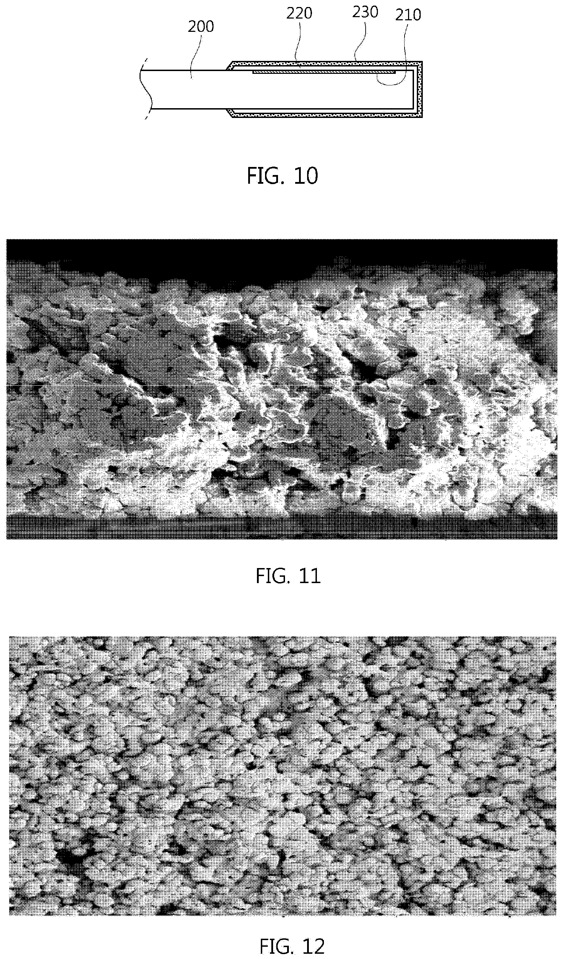

As shown in FIG. 10, when the porous ceramic coating layer 230 is formed on the surface of the sensing part 210 of the oxygen sensor 200 in this way, the thermal shock of the sensing part 210 attributable to high-temperature exhaust gas is reduced, thus preventing the oxygen sensor 200 from being damaged by heat.

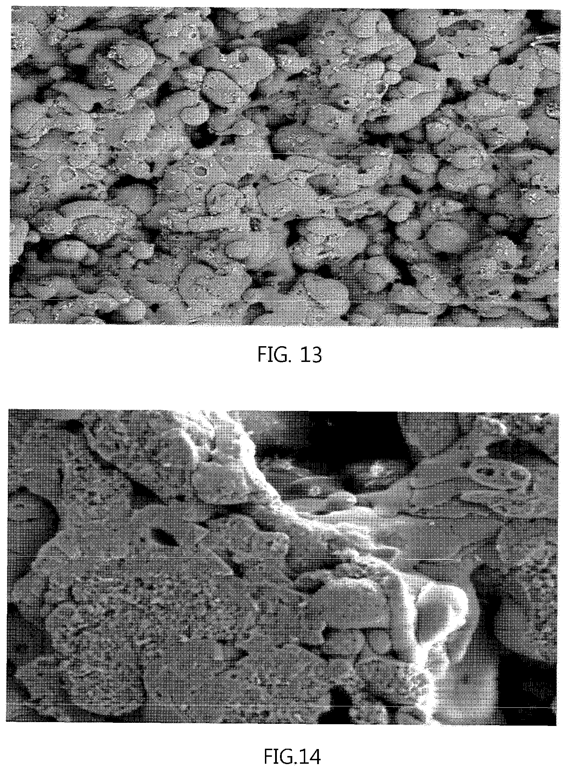

FIG. 11 is an enlarged view of the porous ceramic coating layer of the present invention, which was enlarged by a magnifying power of 339; FIG. 12 is an enlarged view of the porous ceramic coating layer of the present invention, which was enlarged by a magnifying power of 189; FIG. 13 is an enlarged view of the porous ceramic coating layer of the present invention, which was enlarged by a magnifying power of 493; and FIG. 14 is an enlarged view of the porous ceramic coating layer of the present invention, which was enlarged by a magnifying power of 2.48k.

That is, as shown in FIGS. 11 to 14, since the porous ceramic coating layer is formed on the surface of the sensing part 210 of the oxygen sensor, the thermal conductivity between the sensing part 210 and high-temperature exhaust gas is reduced without hindering the sensing part 210 from detecting oxygen concentration, and thus the thermal shock, occurring when water vapor condensed in an exhaust manifold is vaporized by the heater provided in the oxygen sensor 200 in the exhaust gas pipe, is reduced, thereby protecting the sensing part 210.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

1. A method of forming a porous ceramic coating layer on an oxygen sensor, **characterized in that** it comprises the steps of:
(S100) fixing an oxygen sensor (200) in any one of a plurality of fixing brackets (141) provided along one side of a jig (140);
(S200) spraying metal powder onto a sensing part of the oxygen sensor (200) using a plasma gun (120) and simultaneously injecting reaction gas onto the sensing part through a reaction gas injector (130) to form an adhesion layer (220);
(S300) injecting gas into the plasma gun (120) provided with a positive electrode and a negative electrode through a gas inlet and then applying high voltage to the positive electrode and the negative electrode to generate a plasma flame; and
(S400) spraying ceramic powder into the plasma flame through a powder inlet provided at the plasma gun (120) to form a porous ceramic coating layer on the adhesion layer (220).

2. The method of claim 1, wherein the step (S400) of forming the porous ceramic coating layer comprises the steps of:
a) forming the porous ceramic coating layer on the adhesion layer (220) while moving the plasma gun (120) straight to the sensing part of the oxygen sensor (200) fixed in the fixing bracket (141) along one side of the jig (140); and
b) repeatedly performing step a) while rotating the fixing bracket (141) at predetermined angle intervals after step a).

3. The method of claim 2, wherein the step (S400) of forming the porous ceramic coating layer further comprises the steps of:
c) rotating the jig (140) at predetermined angle intervals based on the sensing part (rotation axis) of the oxygen sensor (200) fixed in the fixing bracket (141), when the fixing bracket (141) rotates to an angle of 360° in the step b);
d) repeatedly performing steps a) and b) after step c); and
e) allowing the fixing bracket (141) not to rotate, repeatedly performing step a) and then finishing the step of forming the porous ceramic coating layer, when the rotation axis of the fixing bracket (141) of the jig (140) is flush with the plasma gun (120) after step d).

4. The method of claim 1,
wherein, in the step (S200) of forming the adhesion layer (220), the reaction gas is selected from among O₂, O₃, N₂, H₂, Ar and combinations thereof, and
wherein, in the step (S400) of forming the porous ceramic coating layer, the ceramic powder is selected from among: oxide powder including Y₂O₃, Al₂O₃, ZrO₂, TiO₂ and SiO₂; nitride powder including AlN, Ti and BN; carbide powder including SiC and AlC; and combinations thereof.

5. The method of claim 1, wherein, in the step (S200) of forming the adhesion layer, the adhesion layer (220) has a thickness of 0.1 to 10 µm.

6. The method of claim 1, wherein, in the step (S400) of forming the porous ceramic coating layer, the ceramic powder sprayed through the plasma gun (120) has a particle diameter of 20 to 150 µm.

7. The method of claim 1, wherein, in the step (S400) of forming the porous ceramic coating layer, the porous ceramic coating layer has a thickness of 200 to 500 µm.

## Patentansprüche

1. Verfahren zur Bildung einer porösen Keramikbeschichtung auf einem Sauerstoffsensor, **dadurch gekennzeichnet, dass** er die folgenden Schritte umfasst:
(S100) Befestigung eines Sauerstoffsensors (200) in irgendeinem von mehreren Befestigungswinkeln (141), die entlang einer Seite einer Vorrichtung (140) angebracht sind;
(S200) Aufsprühen von Metallpulver auf das Sensorteil des Sauerstoffsensors (200), unter Verwendung einer Plasma- Spritzpistole (120) und gleichzeitigem Einspeisen von Reaktionsgas durch einen Reaktionsgas - Injektor (130) auf das Sensorteil, um eine haftvermittelnde Schicht (220) zu bilden;
(S300) Einspeisen von Gas in die Plasma- Spritzpistole (120), die mit einer positiven und einer negativen Elektrode versehen ist, durch einen Gaseinlass und dann Anlegen einer hohen Spannung an der positiven Elektrode und der negativen Elektrode, um eine Plasmaflamme zu erzeugen; und
(S400) Sprühen von Keramikpulver durch einen Pulvereinlass, der an der Plasma-Spritzpistole (120) vorgesehen ist, in die Plasmaflamme, um eine poröse Keramikbeschichtung auf der haftvermittelnden Schicht (220) zu bilden.

2. Verfahren nach Anspruch 1, worin der Schritt (S400) der Bildung der porösen Keramikbeschichtung die folgenden Schritte umfasst:
a) Bildung des porösen Keramiküberzugs auf der haftvermittelnden Schicht (220), unter Bewegung der Plasma- Spritzpistole (120) gerade zum Sensorteil des Sauerstoffsensors (200), der in dem Befestigungswinkel (141) entlang einer Seite der Vorrichtung (140) angebracht ist; und
b) wiederholtes Ausführen von Schritt a) unter Drehen des Befestigungswinkels (141) mit vorher festgelegten Winkelintervallen nach Schritt a).

3. Verfahren nach Anspruch 2, worin der Schritt (S400) der Bildung der porösen Keramikbeschichtung weiterhin die folgenden Schritte umfasst:
c) Drehen der Vorrichtung (140) mit vorher festgelegten Winkelintervallen, basierend auf dem Sensorteil (Drehachse) des Sauerstoffsensors (200), der im Befestigungswinkel (141) befestigt ist, wenn sich der Befestigungswinkel (141) in Schritt b) bis zu einem Winkel von 360° dreht.
d) wiederholte Ausführung der Schritte a) und b) nach Schritt c); und
e) zulassen, dass der Befestigungswinkel (141) sich nicht dreht, während wiederholt Schritt a) ausgeführt wird und dann Beendigung des Schritts der Bildung der porösen Keramikbeschichtung, wenn die Drehachse des Befestigungswinkels (141) der Vorrichtung (140) mit der Plasma- Spritzpistole (120) behandelt wird, nach Schritt d).

4. Verfahren nach Anspruch 1,
wobei im Schritt (S200) zur Bildung der haftvermittelnden Schicht (220), das Reaktionsgas aus O₂, O₃, N₂, H₂, Ar und Kombinationen daraus ausgewählt wird und
wobei im Schritt (S400) zur Bildung der porösen Keramikbeschichtung, das Keramikpulver aus Oxidpulver, einschließlich Y₂O₃, Al₂O₃, ZrO₂, TiO₂ und Si0₂; Nitrid - Pulver einschließlich AlN, Ti und BN; Karbid- Pulver einschließlich SiC und AlC; und Kombinationen daraus ausgewählt wird,

5. Verfahren nach Anspruch 1, wobei im Schritt (S200) zur Bildung der haftvermittelnden Schicht, die haftvermittelnde Schicht (220) eine Dicke von 0,1 bis 10 µm hat.

6. Verfahren nach Anspruch 1, wobei im Schritt (S400) zur Bildung der porösen Keramikbeschichtung, die porösie Keramikbeschichtung (120) eine Dicke von 20 bis 150 µm hat.

7. Verfahren nach Anspruch 1, wobei im Schritt (S400) zur Bildung der porösen Keramikbeschichtung, die poröse Keramikbeschichtung (120) eine Dicke von 200 bis 500 µm hat

## Revendications

1. Méthode de formation d'une couche d'enduction poreuse en céramique sur un capteur d'oxygène, **caractérisé en ce qu'**elle comprend les étapes :
(S100) fixation d'un capteur d'oxygène (200) dans l'une quelconque d'une pluralité de crochets de fixation (141) situés le long d'un côté d'un gabarit (140) ;
(S200) pulvérisation d'une poudre de métal sur une zone de détection du capteur d'oxygène (200) en utilisant un canon à plasma (120) et injection simultanée d'un gaz de réaction sur la zone de détection à travers un injecteur de gaz de réaction (130) pour former une couche adhésion (220) ;
(S300) injection de gaz dans le canon à plasma (120) pourvu d'une électrode positive et d'une électrode négative à travers une arrivée de gaz suivi d'une application d'un haut voltage sur l'électrode positive et sur l'électrode négative pour générer une flamme de plasma ; et
(S400) pulvérisation d'une poudre de céramique dans la flamme de plasma à travers une arrivée de poudre fournie au canon à plasma (120) pour former une couche d'enduction poreuse en céramique sur la couche d'adhésion (220).

2. Méthode selon la revendication 1, dans laquelle l'étape (S400) de formation de la couche d'enduction poreuse comprend les étapes :
a) formation de la couche d'enduction poreuse en céramique sur la couche d'adhésion (220) en déplaçant le canon à plasma (120) directement vers la zone de détection du capteur d'oxygène (200) fixé sur les crochets de fixation (141) le long d'un côté du gabarit (140) ; et
b) réitération de manière répétée l'étape a) en tournant le crochet de fixation (141) d'un intervalle angulaire prédéterminé après l'étape a).

3. Méthode selon la revendication 2, dans laquelle l'étape (S400) de formation de la couche d'enduction poreuse en céramique comprend en outre les étapes de :
c) rotation du gabarit (140) à des intervalles angulaires prédéterminés sur la base de la zone de détection (axe de rotation) du capteur d'oxygène (200) fixé dans les crochets de fixation (141), quand le crochet de fixation (141) tourne à un angle de 360° dans l'étape b)
d) réitération de manière répétée les étapes a) et b) après l'étape c) ; et
e) empêchement de tourner du crochet de fixation (141), réitération de manière répétée l'étape a) et ensuite finition de l'étape de formation de la couche d'enduction poreuse en céramique, lorsque l'axe de rotation du crochet de fixation (141) du gabarit (140) est aligné avec le canon à plasma (120) après l'étape d).

4. Méthode selon la revendication 1,
dans laquelle, dans l'étape (S200) de formation de la couche d'adhésion (220), le gaz de réaction est choisi parmi O₂, O₃, N₂, H₂, Ar seul ou en combinaison, et
dans laquelle, dans l'étape (S400) de formation de la couche d'enduction poreuse en céramique, la poudre de céramique est choisie parmi : les oxydes en poudre comprenant Y₂O₃, Al₂O₃, ZrO₂, TiO₂ et SiO₂, les nitrures en poudres comprenant AN Ti et BN, les carbures en poudres comprenant SiC et AlC ; seul ou en combinaison.

5. Méthode selon la revendication 1, dans laquelle, dans l'étape (S200) de formation de la couche d'adhésion, la couche d'adhésion (220) a une épaisseur comprise entre 0,1 et 10 itm.

6. Méthode selon la revendication 1, dans laquelle, dans l'étape (S400) de formation de la couche d'enduction poreuse en céramique, la poudre de céramique pulvérisée à travers le canon à plasma (120) a un diamètre de particule compris entre 20 et 150 itm.

7. Méthode selon la revendication 1, dans laquelle, dans l'étape (S400) de formation de la couche d'enduction poreuse en céramique, la couche d'enduction poreuse en céramique a une épaisseur comprise entre 200 et 500 µm.
